Europäisches Patentamt

European Patent Office

Office européen des brevets

(11) Publication number: **0 106 353**
**B1**

# EUROPEAN PATENT SPECIFICATION

(45) Date of publication of patent specification: **15.07.87**

(21) Application number: **83110348.6**

(22) Date of filing: **17.10.83**

(51) Int. Cl.⁴: **C 07 C 93/04**

(54) **A method for preparing bis(beta-(N,N,-dimethylamino)-ethyl)ether.**

(30) Priority: **18.10.82 JP 181407/82**

(43) Date of publication of application:
**25.04.84 Bulletin 84/17**

(45) Publication of the grant of the patent:
**15.07.87 Bulletin 87/29**

(84) Designated Contracting States:
**DE FR GB IT NL SE**

(56) References cited:
**EP-A-0 005 253**
**GB-A-2 010 839**
**GB-A-2 045 237**

(73) Proprietor: **TOYO SODA MANUFACTURING
CO., LTD.**
**No. 4560, Ooaza Tonda Shinnanyo-shi
Yamaguchi-ken (JP)**

(72) Inventor: **Kumoi, Sadakatsu**
**No. 6-7, Nijigaoko 5-chome
Hikari-shi Yamaguchi-ken (JP)**
Inventor: **Sakka, Hideo**
**No. 2808, Ooaza Tonda
Shinnanyo-shi Yamaguchi-ken (JP)**
Inventor: **Tsutsumi, Yukihiro**
**No. 1723, Ooaza Shimokami
Tokuyama-shi Yamaguchi-ken (JP)**

(74) Representative: **Patentanwälte TER MEER -
MÜLLER - STEINMEISTER**
**Mauerkircherstrasse 45
D-8000 München 80 (DE)**

The file contains technical information
submitted after the application was filed and
not included in this specification

## Description

This invention relates to a method of preparing bis[β-(N,N-dimethylamino)ethyl]ether.

It is known that bis[β-(N,N-dimethylamino)ethyl]ether (hereinafter abbreviated to etheramine) is a very useful catalyst for preparing polyurethanefoam in that it accelerates the reaction between alcohol groups and isocyanate groups and between water and the isocyanate. There are various kinds of processes using miscellaneous starting materials for the manufacture of this compound. Among them, a method utilizing a starting material available at relatively low cost, i.e. a method of producing etheramine by reacting sodium N,N-dimethylaminoethoxide (hereinafter abbreviated to Na-DMAE) derived from N,N-dimethylethanolamine (hereinafter abbreviated to DMEA) with sulfuric anhydride ($SO_3$) as disclosed in GB—A—2 010 839 and GB—A—2 045 237.

The method described in both references comprises basically the following reaction. The starting material, i.e. alkali metal N,N-dialkylaminoalkoxide is dissolved in N,N-dialkylaminoalkanol, the mixed solution in which an organic diluting/dispersing agent added is recycled in a scrubber, $SO_3$-vapor accompanied by nitrogen gas is introduced into the vapor space of a reactor and the first stage reaction is carried out at a relatively low temperature of about 25°C to form an intermediate. Next, as the second stage reaction an etherification reaction is carried out at an elevated temperature of 100 to 140°C. Therefore, the etheramine therefore is produced by a so-called two stage reaction comprising the above first and second stage reactions.

The reactor is designed so as to have an introduction portion for $SO_3$-vapor positioned at the vapor space of the reactor in order to avoid the blocking of the introduction pipe of $SO_3$-vapor caused by burnt matters and solid Glauber's salt produced during the reaction, and it is equipped with a scrubber having a special structure to prevent the stoppage in the passage caused by solid matters. The reaction mixture taken out of the bottom of the reactor is recycled to the top of the scrubber by a pump to be subjected to a vapor-liquid contact reaction.

As organic diluting/dispersing agent mitigating the reaction rate with $SO_3$ by diluting the starting material, aliphatic hydrocarbons having a carbon number of 6—30 and a boiling point of more than 90°C which do not easily react with $SO_3$, are used such as ethers as tetrahydrofuran (boiling point 65—67°C), dioxane, monoglyme, diglyme etc.

Upon reviewing the production of etheramine by the two stage reaction employing paraffins whose effect as organic diluting/dispersing agents is shown in the examples and with blowing $SO_3$-vapor into the liquid phase of the reaction, the inventors have observed the phenomenon that the reaction mixture is discolored to deep-black and deep-black solid matters such as burnt matters etc. adhere as scale on the introduction pipe of $SO_3$-vapor. The reaction yield of etheramine is 65% on basis of $SO_3$ and 61% on basis of DMEA. Accordingly, in case the method described in the references is industrially practiced, a reactor equipped with the scrubber and the attached facilities such as liquid recycling pump etc. is required and the reactor should be equipped with cooling facilities in order to control the reaction temperature at 20—30°C. On the other hand, the recycling of the reaction mixture containing solid matters is required. Furtheron the problem of the adhesion of scale to the vessel wall, the piping path, the scrubber, the pump etc. and the trouble of the stoppage must be feared, because burnt matters resulting during the reaction and the reaction mixture becomes sludge-like because it contains solid matters and Glauber's salt.

Accordingly, a reaction method is desired not requiring special attached facilities such as the scrubber etc., that is, a method for producing etheramine, using available reactors wherein the reaction process comprising blowing $SO_3$-vapor directly into the liquid phase is industrially feasible, and phenomena like scaling of the reaction product to the introduction pipe of $SO_3$-vapor, the stoppage thereof and so on are eliminated and the burnt matters are not formed. Also, from the economical standpoint an increase in the yield of etheramine based on DMEA which is much more expensive than $SO_3$ is strongly desired.

As a result of elaborate studying, the inventors have found the unexpected and novel fact that the yield of etheramine based not only on $SO_3$ but also on DMEA can be greatly improved by performing a one stage reaction at a temperature of below 100°C by introducing $SO_3$-vapor into a reaction mixture comprising dioxane. This invention therefore comprises a method for producing bis[β-(N,N-dimethylamino)ethyl]ether characterized by performing a one stage reaction at a temperature of below 100°C wherein the vapor of sulfuric anhydride is introduced into a mixed solution consisting of sodium N,N-dimethylaminoethoxide, N,N-dimethylethanolamine and dioxane.

Sodium N,N-dimethylaminoethoxide (Na-DMAE) employed in this invention can be produced by reacting N,N-dimethylethanolamine (DMEA) with sodium hydroxide, followed by distilling off the by-product water from the reaction system. The reaction can be practiced in the presence of an azeotropic agent such as toluene and so on. Also, the Na-DMAE can be produced by reacting DMEA with metallic sodium. Instead of Na-DMAE K-DMAE can also be employed as the starting material, but it is not so advantageous being more expensive. The reaction is required to be performed in a state that Na-DMAE is homogeneously dissolved, and therefore the solubility of Na-DMAE, which is dissolved in DMEA and is subjected to the reaction, depends mainly on the mixing ratio with DMEA and the temperature. The amount of DMEA added is desired to be more than the amount which dissolves Na-DMAE homogeneously at the reaction temperature, so that 0.5—3 mol preferably 1—2 mol of DMEA per 1 mol of Na-DMAE are added, though the invention is not particularly limited to these ranges. When using less than 0.5 mol the solubility

2

of Na-DMAE is not enough even at a high reaction temperature region, and even if more than 3 mol is added, there does not arise a particularly advantageous effect in the aspect of yield etc., only increasing the amount of reaction mixture.

In this invention, the addition of an organic solvent is mandatory in order to dilute Na-DMAE and to mitigate the violent reactivity of $SO_3$. As the organic solvent concerned, only dioxane is used. In case of employing other solvents such as pyridine, tri-n-butylamine, tetrahydrofuran and so on, the reaction mixture changes to black, burnt matters cannot be suppressed enough and the increase in the yield of etheramine is not satisfactory.

The reason of increasing the yield of etheramine which is found only in case of adding dioxane is unclear, but it is considered that there is something more than a diluting effect whose main effect lies in mitigating the reaction. The amount of added dioxane added is ordinarily 0.3—3 mol, preferably 0.6—2 mol per 1 mol of Na-DMAE. In case the amount added is less than 0.3 mol, the yield of the product is lowered and unfavourable results of the reaction arise. Also, even though more than 2 mol can be added, there is no favourable effect in further increasing the added amount and it results in only increasing the volume of the reaction mixture. According to this invention, sulfuric anhydride ($SO_3$) can be subjected to the gas-liquid catalytic reaction without any scaling and stoppage of the front outlet of the introduction pipe, even though it is directly introduced into the liquid phase of the reaction mixture as $SO_3$-vapor. Accordingly, it is not necessary at all to adopt a complicated process such as introducing $SO_3$-vapor into the vapor phase portion of the reactor and then practicing the reaction with employing the scrubber, and any conventional reactor can be utilized for the reaction of this invention.

The amount of $SO_3$ is 0.3—0.6 mol, preferably 0.4—0.5 mol per 1 mol of Na-DMAE. When using less than 0.3 mol, lots of the unreacted starting material Na-DMAE remain in the reaction mixture after the reaction and hence it is unfavourable in the aspect of reaction efficiency. In case of adding more than 0.6 mol, the yield of product based on DMEA is lowered, so that this is not favourable.

$SO_3$ is introduced into the reactor as $SO_3$-vapor usually accompanied by nitrogen carrier gas by blowing nitrogen gas into a storage tank of $SO_3$. Accordingly, the supply ratio of nitrogen gas to $SO_3$ relates closely to the temperature of $SO_3$ storage tank and the period of introduction. Generally, the mixed gas having a ratio of 0.1—10 mol of nitrogen gas to 1 mol of $SO_3$ is supplied. The reaction period means the introduction period in other words, and it ranges over 0.5—10 hours. Although it is possible to perform the reaction within 0.5 hour, it is not favourable, because a high amount of nitrogen gas must be supplied as the carrier gas in order to supply $SO_3$ within short period and, as the result, the amount of organic matters escaping with nitrogen gas outside of the reaction system is increasing to enlarge the burden for treating exhaust gas. Although it is possible to perform the reaction over more than 10 hours, this leads to a drop of productivity.

The process of this invention is usually performed at a reaction temperature of lower than 100°C, preferably at 40—100°C in a one stage reaction. Even if the socalled two stage reaction comprising the introduction of $SO_3$ at a low temperature in the region of 20—30°C and, after aging at the same temperature, the reaction at an elevated temperature of 100—140°C as seen in the conventional technique would be applied to the reaction system of this invention, no favourable result can be obtained with respect to yield. The reaction is carried out above 40°C at possible by cooling with water for industrial use and also below about 100°C at the boiling point of dioxane. At a temperature of below 40°C, the increase of yield is not recognized, additional facilities for cooling are required and hence there is little industrial valuable significance. Temperatures above 100°C, are not favourable, because of the drop of yield. If the unreacted Na-DMAE remains in the system after the reaction, enough acid to neutralize it is added for recovering DMEA. The species of the acid is not particularly restricted, but the use of a cheap acid such as sulfuric acid, hydrochloric acid and so on is preferred.

After the completion of the reaction, dioxane, DMEA and etheramine are separated and recovered from the reaction mixture containing much Glauber's salt deposited as by-product. It is possible to recover dioxane, DMEA and etheramine by distillation after adding a nonreactive organic compound having a boiling point of more than 210°C into the reaction mixture as the pot-boiler, but the separating property of Glauber's salt and pot-boiler becomes difficult so this is not an excellent separating and recovering process.

The reaction mixture obtained by the process of this invention is supplied to a down-flow type thin film evaporating apparatus, from the upper portion of which a mixed solution consisting mainly of dioxane, DMEA and etheramine is recovered and is then subjected to rectification, thereby recovering the etheramine with high purity.

As stated above, the process of this invention not only improves the yield of etheramine but also scarcely gives rise to the unfavourable by-reactions such as the occurrence of burnt organic matter, because the reaction mixture after the completion of the reaction is colored only light yellow. It therefore is a method which entirely eliminates the troubles such as the adhesion of scale at the introduction pipe of $SO_3$-vapor etc. located in the reaction mixture and the stoppage due to the solid matters and the like. Accordingly the production of etheramine by means of a universal reactor without requiring a scrubber and the attached facilites such as recycle pump, refrigerating apparatus and the like, becomes feasible by introducing $SO_3$-vapor directly into the reaction mixture, and therefore this invention provides a method of great industrial value in all aspects of apparatus, operativity, economy, productivity and so on.

3

The invention is hereinafter explained in detail by examples, but the method of the invention is not restricted to these examples. The yield of etheramine based on DMEA is calculated as follows.

$$\text{Yield (\%)} = \frac{2 \times [\text{etheramine}]_f}{[\text{Na-DMAE}]_i + [\text{DMEA}]_i - [\text{DMEA}]_f} \times 100$$

[etheramine]$_f$: number of mol of resulting etheramine
[Na-DMAE]$_i$: number of mol of supplied DMAE
[DMEA]$_i$: number of mol of supplied DMEA
[DMEA]$_f$: number of mol of recovered DMEA

Example 1
Into a four neck glass flask equipped with a stirrer, a thermometer, a refluxing condenser and a vapor introduction pipe (the outlet of the vapor introduction pipe which is connected to the supply source of SO$_3$ which is accompanied with nitrogen carrier gas is located near the bottom of the reactor), 166 g of DMEA, 134 g of Na-DMAE and 195 g of dioxane are added and heated with stirring to 50°C. Nitrogen gas is blown into liquid SO$_3$ at a flow rate of 80—200 ml/min, and the mixture of nitrogen gas and SO$_3$ is introduced, by bubbling, into the lower portion of the liquid phase of the reaction system through the vapor introduction pipe. Finally, 44.6 g of SO$_3$ are added over a period of 2 hours and twenty minutes. During this period, the temperature of the reaction is maintained at 50—55°C. The obtained reaction mixture has a light yellow clay color. After the completion of adding SO$_3$, the short column for distillation is connected to the reactor and a single distillation is carried out at a top temperature of 60—100°C under a reduced pressure of 400—13,3 mbar to recover a total weight 443 g of the mixed solution composed of DMEA, dioxane and etheramine. According to gas chromatographic analysis of the recovered solution 67.8 g of etheramine are formed. The yield of etheramine is 76% based on SO$_3$ and also 84% based on DMEA.

Example 2
Into the reactor used in example 1, 166 g of DMEA, 134 g of Na-DMAE and 180 g of dioxane are added and heated with stirring to 80°C. By blowing nitrogen gas into liquid SO$_3$ at a flow rate of 80—200 mol/min, a gas mixture of nitrogen and SO$_3$ is introduced, under bubbling, into the lower portion of the liquid phase of the reaction system through the vapor introduction pipe. Finally, 47.0 g of SO$_3$ are supplied over a period of 4 hours to perform the reaction. During the period, the reaction temperature is kept at 80—85°C. The reaction mixture after the completion of the reaction is slightly brown colored. After the completion of adding SO$_3$, 435 g of a distilled mixed solution consisting mainly of DMEA, dioxane and etheramine is recovered in the same way as in example 1. According to the result of gas-chromatographic analysis of the recovered solution 67.6 g of etheramine are formed. The yield of etheramine is 72% based on SO$_3$, and 79% based on DMEA.

Comparative Example 1
Into the reactor used in example 1, 174 g of DMEA, 134 g of Na-DMAE and 180 g of n-paraffin having a carbon number of 14—15 are added and cooled to 25°C. By blowing nitrogen gas into liquid SO$_3$ at a flow rate of 50—100 ml/min, to a gas mixture of nitrogen and SO$_3$ is introduced, under bubbling, into the lower portion of the liquid phase of the reactor through the vapor introduction pipe. Finally, 44.6 g of SO$_3$ is supplied over a period of 3.5 hours. The reaction temperature during the addition of SO$_3$ is kept at 25—28°C. After completion of adding SO$_3$, the stirring is continued at the same temperature for 30 minutes and whereafter the reaction mixture is heated to 115°C to undergo the reaction for 3 hours. The reaction mixture obtained is colored black. After the completion of the reaction, a short column for distillation is connected to the reactor and a single distillation is carried out at a top temperature of 80—130°C under a reduced pressure of 400—13.3 mbar to recover 274 g of a distillate containing DMEA, etheramine and small amount of n-paraffin. The result of gas-chromatographic analysis of this recovered solution shows that 58.9 g of etheramine was formed. The yield of etheramine is 65% based on SO$_3$ and also 61% based on DMEA.

Comparative Example 2
Into the reactor used in example 1, 174 g of DMEA, 134 g of Na-DMAE and 180 g of n-paraffin having a carbon number of 14—15 are added and heated to 50°C. By blowing nitrogen gas into liquid SO$_3$ at a flow rate of 70—180 ml/min, a gas mixture of nitrogen and SO$_3$ is introduced, by bubbling, into the lower portion of the liquid phase of the reactor through the vapor introduction pipe. Finally, 46.2 g of SO$_3$ are supplied over a period of 4 hours to carry out the reaction. During the period, the reaction mixture is kept at a temperature in the range of 50—55°C. After the completion of adding SO$_3$, 256 g of a distillate consisting of mainly DMEA, etheramine and a small amount of n-paraffin is obtained through the same operation as in comparative example 1. As the result of gas-chromatographic analysis of this distillate, the formation of 48.0 g of etheramine is identified. The yield of etheramine is 52% based on SO$_3$ and also 46% based on DMEA. The result of gas-chromatographic analysis shows that the solid of still residue after the single step distillation which is dissolved into water contains 0.4 g of etheramine. There is observed the adhesion of ·

4

deep black solid precipitates at the outlet of $SO_3$ vapor introduction pipe after the completion of the reaction.

Comparative Examples 3—5

Into the reactor used in example 1, 174 g of DMEA, 134 g of Na-DMAE of 203 g of the additive shown in Table 1 are added and heated to 50°C respectively. By blowing nitrogen gas into liquid $SO_3$ at a flow rate of 70—180 ml/min, the gas mixture of nitrogen and $SO_3$ is introduced, under bubbling, into the lower portion of the liquid phase in the reactor through the vapor introduction pipe. During the period, the reaction mixture is kept at a temperature in the range of 50—55°C. Finally, 46.2 g of $SO_3$ are supplied over a period of 3.5 hours to complete the reaction. The reactor is provided with a column and a single distillation is carried out at a top temperature of 50—100°C under a reduced pressure of 666—13.3 mbar to give a distillate consisting mainly of DMEA, etheramine and the additive. The result of gas-chromatographic analysis of this distillate is shown in Table 1. The gas-chromatographic analysis of the solid of the still residue after the single distillation dissolved into water shows less than 0.1 g of etheramine in every case.

TABLE 1

| Comparative Example | Additive | Yield of etheramine (g) | Yield of etheramine (g) | |
|---|---|---|---|---|
| | | | $SO_3$ base | DMAE base |
| 3 | pyridine | 65.6 | 71 | 61 |
| 4 | tri-n-butyl-amine | 43.4 | 47 | 57 |
| 5 | tetrahydrofuran | 61.9 | 67 | 62 |

## Claims

1. A method for preparing bis[β-(N,N-dimethylamino)ethyl]ether characterized by a one stage reaction at a temperature of not exceeding 100°C comprising introducing the vapor of sulfuric anhydride into a mixed solution consisting of sodium N,N-di-methylaminoethoxide, N,N-dimethylethanolamine and dioxane.

2. The method according to claim 1, characterized in that, N,N-dimethylethanolamine is added in an amount of 0.5—3 mol per 1 mol of sodium N,N-di-methylaminoethoxide.

3. The method according to claim 1, characterized in that, dioxane is added in an amount of 0.3—3 mol per 1 mol of sodium N,N-dimethylaminoethoxide.

4. The method according to claim 1, characterized in that sulfuric anhydride ($SO_3$) is introduced as such or in the form of a mixture with nitrogen gas.

5. The method according to claim 1, characterized in that sulfuric anhydride ($SO_3$) is introduced in an amount of 0.3—0.6 mol per 1 mol of sodium N,N-dimethylaminoethoxide.

6. The method according to claim 4, characterized in that sulfuric anhydride ($SO_3$) is introduced in the form of a mixture with 0.1—10 mol of nitrogen gas per 1 mol of $SO_3$.

7. The method according to claim 4, characterized in that sulfuric anhydride ($SO_3$) is being introduced over a period of 0.5—10 hours.

## Patentansprüche

1. Verfahren zur Herstellung von Bis[β-(N,N-dimethylamino)-ethyl]ether, gekennzeichnet durch eine Einstufenreaktion, die bei einer Temperatur von nicht mehr als 100°C durchgeführt wird, wobei Schwefeltrioxiddampf in eine aus Natrium-N,N-dimethylaminoethoxid, N,N-dimethylethanolamin und Dioxan bestehende gemischte Lösung geleitet wird.

2. Verfahren nach Anspruch 1, dadurch gekennzeichnet, daß das N,N-Dimethylethanolamin in einer Menge von 0,5—3 Mol pro einem Mol Natrium-N,N-dimethylaminoethoxid zugefügt wird.

3. Verfahren nach Anspruch 1, dadurch gekennzeichnet, daß das Dioxan in einer Menge von 0,3—3 Mol pro Mol Natrium-N,N-dimethylaminoethoxid zugefügt wird.

4. Verfahren nach Anspruch 1, dadurch gekennzeichnet, daß das Schwefeltrioxid ($SO_3$) entweder als solches oder in Form einer Mischung mit gasförmigem Stickstoff eingeleitet wird.

5. Verfahren nach Anspruch 1, dadurch gekennzeichnet, daß das Schwefeltrioxid ($SO_3$) in einer Menge von 0,3—0,6 Mol pro Mol Natrium-N,N-dimethylaminoethoxid eingeleitet wird.

6. Verfahren nach Anspruch 4, dadurch gekennzeichnet, daß das Schwefeltrioxid ($SO_3$) in Form einer Mischung mit 0,1—10 Mol gasförmigem Stickstoff pro Mol $SO_3$ eingeleitet wird.

7. Verfahren nach Anspruch 4, dadurch gekennzeichnet, daß das Schwefeltrioxid (SO$_3$) über einen Zeitraum von 0,5—10 Stunden eingeleitet wird.

**Revendications**

1. Procédé de préparation d'éther bis[β-(N,N-diméthylamino)éthylique] caractérisé par une seule étape de réaction à une température ne dépassant pas 100°C comprenant l'introduction de vapeur d'anhydride sulfurique dans une solution mixte constituée de N,N-diméthylaminoéthylate de sodium, de N,N-diméthyléthanolamine et de dioxanne.

2. Procédé selon la revendication 1, caractérisé en ce qu'on ajoute la N,N-diméthyléthanolamine en une quantité de 0,5 à 3 moles pour 1 mole de N,N-diméthylaminoéthylate de sodium.

3. Procédé selon la revendication 1, caractérisé en ce qu'on ajoute le dioxanne en une quantité de 0,3—3 moles pour 1 mole de N,N-diméthylaminoéthylate de sodium.

4. Procédé selon la revendication 1, caractérisé en ce qu'on introduit l'anhydride sulfurique (SO$_3$) tel quel ou sous forme d'un mélange avec de l'azote gazeux.

5. Procédé selon la revendication 1, caractérisé en ce qu'on introduit l'anhydride sulfurique (SO$_3$) en une quantité de 0,3—0,6 mole pour 1 mole de N,N-diméthylaminoéthylate de sodium.

6. Procédé selon la revendication 4, caractérisé en ce qu'on introduit l'anhydride sulfurique (SO$_3$) sous forme d'un mélange avec 0,1—10 moles d'azote gazeux pour 1 mole de SO$_3$.

7. Procédé selon la revendication 4, caractérisé en ce qu'on introduit l'anhydride sulfurique (SO$_3$) en une période de 0,5—10 heures.